# EUROPÄISCHE PATENTSCHRIFT

(11) **EP 3 861 925 B1**
(45) Veröffentlichungstag und Bekanntmachung des Hinweises auf die Patenterteilung: **01.11.2023**
(21) Anmeldenummer: 20155560.4
(22) Anmeldetag: 05.02.2020
(51) Int. Cl.: A61B 5/00

(54) **KLOBRILLE MIT EINER VORRICHTUNG ZUM ERFASSEN VON WERTEN UND METHODE ZUR VERWENDUNG DAVON**
TOILET SEAT WITH A DEVICE FOR DETECTING VALUES AND METHOD OF USE THEREOF
LUNETTE DE TOILETTES DOTÉE D'UN DISPOSITIF DE DÉTECTION DE VALEURS ET SON PROCÉDÉ D'UTILISATION

(43) Veröffentlichungstag der Anmeldung: 11.08.2021
(73) Patentinhaber: Herbst, Martin, 5090 Lofer (AT)
(72) Erfinder: Herbst, Martin, 5090 Lofer (AT)
(74) Vertreter: Grünecker Patent- und Rechtsanwälte PartG mbB

(56) Entgegenhaltungen:
- CN-A- 109 793 501
- DE-A1-102015 112 819
- US-A1- 2012 253 224
- US-A1- 2018 085 098
- US-A1- 2018 303 466
- US-B1- 9 756 297

## Beschreibung

Die Erfindung betrifft eine Klobrille mit einer Vorrichtung zum Messen von Werten, ein System zum Erfassen von Werten, das eine solche Klobrille umfasst, sowie ein Verfahren zum Erfassen von Werten eines Nutzers mittels einer solchen Klobrille oder eines solchen Systems.

Klobrillen werden von jedem täglich mehrmals verwendet. Es ist bekannt, solche Klobrillen mit Gewichtssensoren, Sensoren zur Messung des Fettgehalts, Wassergehalts und Eiweißgehalts von Gewebe auszustatten, um Körperwerte eines Nutzers zu ermitteln, beispielsweise aus der EP 3 417 766 A1. Somit kann eine Überprüfung der entsprechenden Werte jedes Mal erfolgen, wenn der Nutzer die Toilette benutzt, sodass ein regelmäßiges Überprüfen der Werte gewährleistet ist, ohne dass der Nutzer zusätzliche Wege und Aufwand auf sich nehmen muss, um die Messungen durchzuführen. Durch die ermittelten Daten lässt sich der körperliche Zustand und die Fitness des Nutzers überwachen. Eine einzige Klobrille kann zudem normalerweise von mehreren und grundsätzlich beliebig vielen Nutzern verwendet werden. Weiterer relevanter Stand der Technik ist durch die folgenden Dokumente offenbart: US 2018/085098 A1, US 2018/303466 A1 und US 9 756 297 B1.

Ausgehend davon stellt sich nun die Aufgabe, eine verbesserte Klobrille anzugeben, die insbesondere eine genauere und/oder kostengünstige Bestimmung des Gesundheitszustandes des Nutzers ermöglicht.

Diese Aufgabe wird gelöst durch eine Klobrille nach Anspruch 1, ein System nach Anspruch 5 und ein Verfahren nach Anspruch 7. Weitere Ausführungsformen werden in den abhängigen Ansprüchen beschrieben.

Eine Klobrille umfasst eine Vorrichtung zum Erfassen von Werten, insbesondere gesundheitsbezogenen Werten eines Nutzers. Die Vorrichtung zum Erfassen von Werten umfasst eine Multispektralkamera zum Erfassen von Multispektraldaten.

Als gesundheitsbezogene Werte eines Nutzers werden in diesem Text Werte bezeichnet, von denen jeder allein und die Gesamtheit dieser Werte einen Rückschluss auf den Gesundheitszustand eines Nutzers zulassen. Diese gesundheitsbezogenen Werte können Werte umfassen oder sein, die nicht direkt im oder am Körper des Nutzers gemessen werden, aber Rückschlüsse auf den Körper des Nutzers zulassen, beispielsweise Messwerte von Ausscheidungen, Abfallstoffen, Ausdünstungen, Haaren und/oder Nägeln. Gesundheitsbezogene Werte können alternativ oder zusätzlich Werte umfassen oder sein, die direkt am Körper des Nutzers gemessen werden, beispielsweise Körperkennwerte wie die Temperatur, Herzschlagrate, Fettgehalt des Körpers, Eiweißgehalt des Körpers, Wassergehalt des Körpers, Sauerstoffsättigung des Blutes und/oder andere Körperkennwerte.

Multispektralkameras können durch Erfassen von Licht bzw. Strahlung in verschiedenen Spektralbereichen (also Licht bzw. Strahlung in verschiedenen Frequenzbereichen) Multispektraldaten liefern. Solche Multispektraldaten können durch die Information in verschiedenen Spektralbereichen (Frequenzbereichen) eine Information über Bestandteile und vorhandene Moleküle in einem Material liefern, von dem kommendes Licht durch die Multispektralkamera erfasst wurde. Mit solchen Multispektraldaten kann somit eine Analyse der jeweils aufgenommenen Stoffe erfolgen. Eine Multispektralkamera kann beispielsweise Multispektraldaten in mindestens zwei, beispielsweise zwei, drei oder vier, Spektralbereichen erfassen, wobei jeder Spektralbereich ein kontinuierliches Spektrum, z.B. von mindestens 20 nm oder mindestens 50 nm, umfassen kann.

Eine Multispektralkamera kann Licht bündeln und dann die Multispektraldaten von der Gesamtheit des gebündelten Lichtes erfassen oder Multispektraldaten in einem zwei- oder dreidimensionalen Bild erfassen, wobei jeweils Multispektraldaten für jeden Punkt (oder anderen vorher definierten Bereich) des zwei- bzw. dreidimensionalen Bildes erfasst werden können.

Die Multispektralkamera kann insbesondere Multispektraldaten erfassen (und somit für die Weiterverwendung erzeugen). Diese Multispektraldaten können Informationen über reflektierte oder emittierte elektromagnetische Strahlung unterschiedlicher Wellenlängen enthalten. Die Multispektralkamera kann verschiedene Spektren erfassen, beispielsweise das komplette sichtbare Lichtspektrum (oder einen Teilbereich des sichtbaren Lichtspektrums) und/oder nicht sichtbare Lichtspektren, z.B. Ultraviolett oder Infrarot, und/oder Spektren, die über ultraviolette oder infrarote Spektren hinausgehen. Die Multispektralkamera kann beispielsweise die Lichtmenge, die von verschiedensten Stoffen, wie etwa Bakterien oder Molekülen, reflektiert und/oder absorbiert wird, erfassen (und messen), so dass diese danach analysiert werden kann. Die Multispektralkamera kann einen oder mehrere Sensor(en) umfassen, welche(r) das von den Stoffen reflektierte oder emittierte Licht in verschiedenen Wellenlängen auffangen kann/können.

Mit der Multispektralkamera kann optional zusätzlich zu den Multispektraldaten auch ein konventionelles (zweidimensionales) Bild aufgenommen werden. Ein solches Bild kann dann z.B. durch automatische Bildanalyse, ausgewertet werden. So kann z.B. das Erscheinungsbild von Ausscheidungen mitberücksichtigt werden. Alternativ können in der Vorrichtung zum Erfassen von Werten eine, zwei oder mehr weitere Kameras umfasst sein, die ein konventionelles (zweidimensionales) Bild aufnehmen können.

Die Multispektralkamera kann hinsichtlich der zu erfassenden Spektren (insbesondere hinsichtlich der verwendeten Frequenzen) auf die untersuchten Materialien, insbesondere nachzuweisende Moleküle, abgestimmt sein. Die Multispektralkamera kann so ausgebildet sein, dass die von ihr erfassten Spektralbereiche diejenigen Spektralbereiche sind, bei denen die nachzuweisenden Moleküle oder untersuchten Materialien typische Spektren aufweisen. Nachzuweisende Moleküle können beispielsweise Blutbestandteile, Blut, Bestandteile von Urin, Bestandteile von Haut o. ä. sein oder umfassen. Somit kann insbesondere eine Früherkennung von Warnzeichen, wie Übersäuerung des Körpers oder Entzündungszeichen und/oder eine Belastung mit Umweltgiften, z.B. Schwermetallen oder Medikamentenresten, und/oder eine Früherkennung von Krankheiten, wie z.B. Darmkrebs oder anderen Krebsformen, erfolgen.

Die Spektren der Multispektralkamera können durch mechanische und/oder elektronische Filter so eingestellt sein oder werden, dass mit den erfassten Spektren ein Rückschluss über das Vorhandensein oder Abwesenheit der untersuchten Materialien oder nachzuweisenden Moleküle möglich ist und/oder eine quantitative Bestimmung der Menge der nachzuweisenden Moleküle erfolgen kann. Eine solche Multispektralkamera kann hinsichtlich der verwendeten Spektren (Frequenzen) ansteuerbar sein und/oder mit verschiedenen Filtern verwendbar sein.

Die Multispektralkamera kann einen (mechanischen oder elektronischen) Filter umfassen, der das Spektrum von Wasser entfernt, so dass dieser (Haupt-)Bestandteil des Körpers und körpereigenen Stoffen nicht als Störsignal auftritt.

Die Multispektralkamera kann in der Klobrille beweglich angeordnet sein, sodass der von ihr erfasste Bereich (also der Bereich, aus dem Licht auf die Multispektralkamera fallen kann) geändert werden kann. In anderen Ausführungsformen ist sie in der Klobrille fest angeordnet, so dass sie einen vorher bestimmten Bereich (also Licht daraus) erfassen kann.

Sie kann mit dem Umgebungslicht oder in der Toilette oder anderen Stellen vorhandenen Lichtquellen arbeiten. Eine separate Lichtquelle für die Multispektralkamera ist hinter einem anderen Fenster als die Multispektralkamera in der Klobrille angeordnet. Eine in der Klobrille umfasste Lichtquelle kann insbesondere ein oder mehrere Spektralbereich(e) abdecken, beispielsweise Infrarot, UV und/oder spektrales Licht (z.B. sichtbares Licht oder ein Teilbereich aus dem sichtbaren Licht). Das Spektrum einer in der Klobrille umfassten Lichtquelle kann durch Ansteuerung der Lichtquelle anpassbar (wählbar) sein. Alternativ können eine oder mehrere Lichtquellen für bestimmte Spektralbereich(e) in der Klobrille umfasst sein. Die Lichtquelle kann fest angeordnet sein, oder alternativ kann die Lichtquelle bewegbar sein, so dass die Richtung des von ihr ausgestrahlten Lichts festgelegt werden kann.

Eine solche Klobrille kann mit einem Standardanschluss ausgestattet sein, sodass sie auf handelsüblichen Toiletten nachgerüstet werden kann.

Die Klobrille kann einen Akku zur Versorgung der Vorrichtung zum Erfassen von Werten umfassen. Alternativ oder zusätzlich kann sie einen Stromanschluss umfassen und/oder eine andere Möglichkeit, die darin umfasste Vorrichtung zum Erfassen von Werten mit Strom zu versorgen, z.B. durch Aufladen eines Akkus. Beispielsweise kann sie einen Ladeanschluss wie beispielsweise eine USB, Mini-USB oder andere Ladebuchse umfassen und/oder einen konventionellen Stromanschluss, der an das Stromnetz angeschlossen werden kann. Alternativ kann die Klobrille so ausgebildet sein, dass ein Akku in der Klobrille induktiv geladen werden kann. Eine solche Klobrille kann auch mit einer dazu passenden Toilette ausgeliefert werden oder für eine dazu passende Toilette vorgesehen sein. In solchem Fall kann die Stromversorgung der Klobrille beispielsweise durch einen Stromanschluss der Toilette erfolgen und beispielsweise die Stromzufuhr zur Klobrille über die Verbindungsstücke zur Toilette erfolgen.

Des Weiteren kann die Klobrille eine Übertragung von Daten, insbesondere erfassten Werten, an einen Prozessor außerhalb der Klobrille ermöglichen, beispielsweise über Kabel, wie z.B. ein USB-Kabel oder Netzwerkkabel und/oder über einen drahtlosen Anschluss, beispielsweise WiFi oder Bluetooth.

Eine erfindungsgemäße Klobrille kann einen Toilettendeckel umfassen. Der Toilettendeckel kann an der Klobrille angeordnet sein und diese bei Nichtgebrauch schützen. Der Toilettendeckel oder die Klobrille kann eine Vorrichtung zur Reinigung der Klobrille umfassen, so dass die Klobrille mit der Vorrichtung zum Erfassen von Werten oder Teile davon nach jeder Benutzung gereinigt werden können, und für eine weitere Benutzung zur Verfügung stehen. Die Multispektralkamera ist in der Klobrille hinter einem Fenster angeordnet. Sie kann daher durch das Fenster von außen sichtbar sein. Das Fenster kann in den Spektralbereichen, in denen nachzuweisende Moleküle typische Spektren aufweisen, lichtdurchlässig sein. Das Fenster lässt mindestens 80% des Lichts in den betrachteten Spektralbereichen passieren. Eine Lichtquelle ist hinter einem anderen Fenster angeordnet.

Ein solches Fenster in der Klobrille kann säurebeständig sein, so dass es mit handelsüblichen Chemikalien zur Toilettenreinigung gereinigt werden kann. Es kann bündig mit der Klobrille abschließen, so dass sich zwischen Klobrille und Fenster kein Zwischenraum bildet, in dem sich Schmutz ablagern kann.

In einer Klobrille ist die Multispektralkamera zum Erfassen von Multispektraldaten von Ausscheidungen ausgerichtet oder kann so ausgerichtet werden. Insbesondere kann die Multispektralkamera so ausgerichtet sein oder werden, dass Multispektraldaten des Inhalts der Toilettenschüssel erfasst werden können. Beispielsweise kann die Multispektralkamera an der Unterseite der Klobrille und/oder an der Innenseite der Klobrille angeordnet sein. Bei Angabe von Ober-, Unter-, Innen-und Außenseite der Klobrille wird hierbei angenommen, dass die Klobrille sich in einem benutzungsbereiten Zustand befindet, also auf der Toilette aufliegt, wobei die Oberseite dem Nutzer zugewandt ist, die Unterseite der Toilettenschüssel zugewandt ist, die Außenseite seitlich an der Klobrille der Toilette abgewandt angeordnet ist und die Innenseite der Klobrille die Öffnung begrenzt, durch welche die Toilette benutzt werden kann. Die Klobrille kann so ausgebildet sein, dass sie dazu geeignet ist, mit der Unterseite auf der Toilettenschüssel aufzuliegen und jeweils nach innen etwas über den Rand der Toilettenschüssel überzustehen, also insbesondere nicht bündig damit abzuschließen, beispielsweise mindestens 0,5 cm, beispielsweise mindestens 1 cm oder mindestens 2 cm, nach innen über den Rand der Toilettenschüssel überzustehen.

Die Multispektralkamera kann insbesondere an der Unterseite der Klobrille angeordnet sein, z.B. in einem Bereich, der an die Innenseite der Klobrille angrenzt. Bei einer solchen Anordnung der Multispektralkamera in einer Klobrille, die dazu ausgebildet ist, nach innen über den Rand der Toilettenschüssel überzustehen, kann so erreicht werden, dass die Multispektralkamera nicht durch den Rand der Toilettenschüssel verdeckt wird.

Eine Multispektralkamera, die zum Erfassen von Multispektraldaten von Ausscheidungen ausgerichtet sein oder werden kann, kann insbesondere hinsichtlich der zu erfassenden Spektren auf zu untersuchende Moleküle oder Materialien abgestimmt sein oder werden. Solche zu untersuchenden Materialien und Moleküle können z.B. umfassen Blut, Bestandteile von Blut, Krebszellen bzw. deren Residuen, Farbe und/oder andere Rückstände in Ausscheidungen. Die Multispektralkamera kann insbesondere dazu ausgebildet sein, Spektralbereiche zu untersuchen, in denen die entsprechenden Moleküle oder Materialien typische Spektren aufweisen, z.B. durch einen oder mehrere Filter. Die Verwendung einer Multispektralkamera, die zum Erfassen von Multispektraldaten von Ausscheidungen ausgerichtet sein oder werden kann, hat den Vorteil, dass - anders als bei herkömmlichen Verfahren mittels Teststreifen oder Laboranalyse - kein Verbrauchsmaterial entsorgt werden muss, obwohl Ausscheidungen umfassend untersucht werden können.

Die Multispektralkamera ist zum Erfassen von Multispektraldaten des Körpers des Nutzers ausgerichtet oder kann so ausgerichtet werden. Beispielsweise kann sie an der Oberseite der Klobrille hinter einem Fenster so angeordnet sein, dass Multispektraldaten des Körpers des Nutzers erfasst werden können, während dieser auf der Klobrille sitzt. Die Lichtquelle kann in einem solchen Fall optional von der Kamera umfasst und/oder hinter dem gleichen Fenster wie die Kamera angeordnet sein.

Alternativ kann die zum Erfassen von Multispektraldaten des Körpers des Nutzers ausgerichtete oder ausrichtbare Multispektralkamera an der Innenseite der Klobrille angeordnet sein, sodass die Daten des Körpers des Nutzers durch Aufnahme schräg nach oben erfasst werden können, während der Nutzer auf der Klobrille sitzt. Bei Anordnung an der Innenseite der Klobrille kann die Multispektralkamera beweglich angeordnet (und ansteuerbar) sein, sodass sowohl Multispektraldaten der Ausscheidungen als auch Multispektraldaten des Körpers des Nutzers mit ein und derselben Multispektralkamera erfasst werden können. Diese Multispektralkamera kann auch hinsichtlich der verwendeten Spektren anpassbar sein, beispielsweise mit elektronischen und/oder mechanischen Filtern ansteuerbar sein, so dass sie für verschiedene zu untersuchende Materialien und nachzuweisende Moleküle eingestellt werden kann.

Insbesondere kann die Multispektralkamera so ausgerichtet sein oder werden, dass Multispektraldaten der Haut und durch die Haut des Nutzers erfasst werden können, beispielsweise Multispektraldaten, die die Hautfarbe und Hautbeschaffenheit, oder die Sauerstoffsättigung des Blutes o. ä beschreiben. Eine solche Multispektralkamera, die zum Erfassen von Multispektraldaten des Körpers des Nutzers ausgerichtet ist oder werden kann, kann hinsichtlich der zu untersuchenden Materialien und Moleküle abgestimmt sein, beispielsweise auf Spektren, die auf Sauerstoffsättigung oder andere Bestandteile von Blut oder Haut Rückschlüsse erlauben.

In der Klobrille kann die Vorrichtung zum Erfassen eine erste und eine zweite Multispektralkamera zum Erfassen von Multispektraldaten umfassen. Jede dieser beiden Multispektralkameras kann wie zuvor beschrieben ausgebildet sein. Beispielsweise können eine oder beide Multispektralkameras (jeweils oder gemeinsam) hinter einem Fenster angeordnet sein. Die erste Multispektralkamera kann zum Erfassen von Multispektraldaten der Ausscheidungen ausgerichtet sein oder ausgerichtet werden, und so ausgebildet sein, wie zuvor für eine Multispektralkamera zum Erfassen von Multispektraldaten der Ausscheidungen beschrieben. Die zweite Multispektralkamera kann zum Erfassen von Multispektraldaten des Körpers eines Nutzers ausgerichtet sein oder werden und kann wie oben für eine solche Multispektralkamera beschrieben ausgebildet und angeordnet sein.

Die Vorrichtung zum Erfassen von Werten eines Nutzers kann einen Sensor zum Erfassen der Hautfarbe eines Nutzers umfassen, z.B. einen Spektralsensor, der dazu geeignet ist, die Hautfarbe des Nutzers zu bestimmen. Diese Information kann dann dazu verwendet werden, die Intensität und spektrale Emission einer Lichtquelle für weitere Erfassung von Werten eines Nutzers durch die Multispektralkamera anzupassen.

Die Vorrichtung zum Erfassen von Werten eines Nutzers kann alternativ oder zusätzlich einen Hautoberflächendetektor umfassen. Ein Hautoberflächendetektor kann zum Beispiel zum Messen von Partikeln und Ausdünstungen auf der Haut ausgebildet sein, beispielsweise zum Messen der elektrodermalen Aktivität der Haut und/oder der Schweißmenge auf der Haut.

Die Vorrichtung zum Erfassen von Werten eines Nutzers kann alternativ oder zusätzlich ein Laserspektrometer umfassen. Ein Laserspektrometer kann beispielsweise zur Untersuchung von atomaren oder molekularen Bestandteilen eingesetzt werden und beispielsweise Fingerabdrücke von molekularen Zusammensetzungen biologischer Proben jeglicher Art erstellen. Solche Fingerabdrücke von molekularen Zusammensetzungen biologischer Proben können insbesondere erlauben, festzustellen, ob bestimmte (bekannte) atomaren oder molekularen Bestandteile vorhanden sind und optional, in welcher Menge oder Konzentration diese Bestandteile vorhanden sind.

Die Vorrichtung zum Erfassen von Werten kann einen Gassensor umfassen, mit dem beispielsweise weitere Informationen über die Ausscheidungen, insbesondere deren Ausgasung, oder Ausgasungen aus der Haut oder Gasgemische aus der Umgebungsluft gewonnen werden können.

Der Gassensor kann ein Chemosensor sein für die Detektion gasförmiger Substanzen. Beispielsweise kann mit dem Chemosensor der Anteil bestimmter chemischer Inhaltsstoffe des Gases in ein elektrisches Signal umgewandelt werden, welches ausgelesen werden kann (und somit als Wert erfasst werden kann, der z.B. bei der Bestimmung von ein oder mehrere Parameter zum Gesundheitszustand des Nutzers berücksichtigt werden kann). Der Gassensor kann Einschränkungen bei der Selektivität und Reaktion auf eine oder mehrere Zielsubstanzen aufweisen. Die Sensorselektivität kann beispielsweise mit einer bestimmten Reaktivität auf eine, zwei oder mehr Zielsubstanzen voreingestellt sein (wobei der Sensor also beispielsweise selektiv ein, zwei oder mehr Zielsubstanzen erkennt und bei anderen Substanzen keine (relevante) Reaktion zeigen kann).

Alternativ kann die Sensorsensibilität variierbar sein (z.B. durch Ansteuerung des Sensors). Die Messungen können beispielsweise durch elektrische und/oder mechanische Sensoren erfolgen. Der Gassensor kann optional ein molekularer Sensor sein. Der Gassensor kann ein aktiver oder ein passiver Sensor sein.

Der Gassensor kann ein CO2eq (CO2 äquivalent) ausgeben, welches rechnerisch Schlüsse aus der H2-Konzentration abbilden kann.

Die Vorrichtung zum Erfassen von Werten kann alternativ oder zusätzlich einen oder mehrere weitere Sensoren, beispielsweise einen oder mehrere Gewichtssensoren, Messungssensoren für die Messung des Hautwiderstands (beispielsweise zur Bestimmung des Körperfettanteils), einen Sensor zur Messung des Wassergehalts von Gewebe, einen Sensor zur Messung der Knochenmasse, einen Schadstoffsensor, einen Hochfrequenzsensor oder Sensoren für andere Frequenzen, einen Röntgensensor, einen Sensor oder ein Sensorsystem zur Analyse des vegetativen Nervensystems, einen Temperatursensor, einen Sensor zur Messung des Herzschlags und/oder einen Messsensor für Blutdruck, EKG, einen Sensor zur Messung des Blutzuckers, einen Sensor zur Messung des Blutstroms und/oder Blutvolumens, einen Sensor zur Messung von Laktat und/oder der Salzkonzentration im Blut umfassen. Die Vorrichtung kann Sensoren oder Messgeräte umfassen, die einen Geigerzähler oder anderen Strahlungssensor umfassen zur Messung von Radioaktivität in Ausscheidungen und/oder Körper des Nutzers. Jeder solcher Sensor kann dazu beitragen, die Gewinnung eines noch genaueren Bildes des Gesundheitszustands eines Nutzers zu ermöglichen. Die Vorrichtung zum Erfassen von Werten kann zudem einen Biofeedbacksensor umfassen.

Die Klobrille kann ein Display umfassen, das die Anzeige von Informationen, beispielsweise einfache Informationen oder Warnhinweisen und/oder Biofeedback ermöglicht.

Die Erfindung umfasst des Weiteren ein System zum Erfassen von Werten eines Nutzers, das eine Klobrille wie zuvor beschrieben umfasst und einen Prozessor oder ein Prozessorsystem aus zwei oder mehr Prozessoren, wobei der Prozessor oder das Prozessorsystem dazu konfiguriert ist, unter Verwendung von Multispektraldaten ein oder mehrere Parameter zum Gesundheitszustand des Nutzers zu bestimmen und ein Ergebnis auszugeben (der Prozessor oder das Prozessorsystem werden im Folgenden in ihrer Gesamtheit auch als Auswertprozessor bezeichnet).

Der Prozessor oder ein Prozessor des Prozessorsystems können in der Klobrille angeordnet sein. Der Prozessor in der Klobrille kann dazu konfiguriert sein, die Messwerte der Vorrichtung zum Erfassen von Werten aufzunehmen, zwischenzuspeichern und nach außen zu transferieren, beispielsweise mittels Kabel oder kabellos.

Der Prozessor oder ein oder mehrere Prozessoren des Prozessorsystems können alternativ oder zusätzlich außerhalb der Klobrille, z.B. in einem Computer in der Nähe, in der Cloud, in einem Cloudsystem, einem dezentralen System, einem Smartphone oder einem anderen mobilen Endgerät angeordnet sein.

Der eine oder die mehreren Parameter zum Gesundheitszustand des Nutzers können insbesondere die Multispektraldaten oder ein gemessener Wert direkt oder ein aus den Multispektraldaten und optional weiteren gemessenen Werten errechneter oder anders abgeleiteter Wert sein. Insbesondere können zur Bestimmung des einen oder der mehreren Parameter zum Gesundheitszustand des Nutzers Informationen aus den Multispektraldaten von einer oder mehr Multispektralkameras gewonnen werden. Beispielsweise kann der Auswertprozessor konfiguriert sein, um algorithmisch aus den Multispektraldaten Informationen, z.B. über einen (oder mehrere) Bestandteil(e) von untersuchten Materialien und/oder über nachzuweisende Moleküle, zu bestimmen. Dazu kann er insbesondere dazu konfiguriert sein, Hintergrundspektren (z.B. Wasser und/oder Reflektionen der Toilettenschüssel) oder andere Störsignale aus den Multispektraldaten herauszurechnen und/oder durch Betrachtung und Vergleich der Multispektraldaten in bestimmten Spektralbereichen Informationen über Vorhandensein oder Abwesenheit bestimmter Bestandteil(e) und/oder nachzuweisender Moleküle zu gewinnen. Diese Informationen über einen oder mehrere Bestandteil(e) von untersuchten Materialien und/oder über nachzuweisende Moleküle können einen oder mehrere Parameter zum Gesundheitszustand des Nutzers darstellen.

Alternativ oder zusätzlich können zur Bestimmung des einen oder der mehreren Parameter zum Gesundheitszustand des Nutzers zuvor beschriebenen Informationen aus den Multispektraldaten (z.B. über einen oder mehrere Bestandteil(e) von untersuchten Materialien und/oder über nachzuweisende Moleküle) mit optional vorhandenen einem oder mehreren weiteren Messdaten aus einem oder mehreren anderen Sensoren und/oder mit optional vorhandenen einer oder weiteren Informationen über den Nutzer, z.B. Alter, Größe, medizinische Daten o.ä. kombiniert werden. Daraus können dann ein oder mehrere Parameter zum Gesundheitszustand bestimmt werden, z.B. durch Berechnung oder Vergleich mit Standardwerten. Diese können optional mit Daten des Nutzers aus der Vergangenheit verglichen werden, um so einen Trend festzustellen. Der Auswertprozessor kann konfiguriert sein, um einen, mehrere oder alle der zuvor hinsichtlich der Auswertung beschriebenen Schritte durchzuführen.

Der Auswertprozessor ist konfiguriert, ein Ergebnis auszugeben. Das Ergebnis können der eine oder mehrere Parameter zum Gesundheitszustand des Nutzers sein, diese umfassen oder darauf basieren.

Das Ergebnis kann auf einem an den Auswertprozessor angeschlossenen oder ansteuerbaren Display, z.B. Monitor, angezeigt werden, an einen oder mehrere Empfänger in Text- oder maschinenlesbarer Form versendet, per Sprachausgabe ausgegeben oder an einen anderen Prozessor, z.B. den Prozessor in der Klobrille oder ein Smartphone übersendet oder in anderer Weise ausgegeben werden. Beispielsweise kann ein Ergebnis z.B. auf dem Display der Klobrille angezeigt werden und/oder an einem anderen Gerät, z.B. dem Smartphone des Benutzers angezeigt werden.

Beispielsweise kann der Auswertprozessor dazu konfiguriert sein, basierend auf den Werten ein binäres Gesamtergebnis anzugeben, bei dem entweder ausgegeben wird, dass alles in Ordnung ist oder dass die Messwerte auf ein Problem hindeuten. Alternativ kann der Auswertprozessor dazu konfiguriert sein, ein detaillierteres Ergebnis, z.B. mehrere Parameter zum Gesundheitszustand des Nutzers, einen Vergleich mit Daten des Nutzers aus der Vergangenheit, einen Trend hinsichtlich der Daten, einen Biognosewert des Nutzers oder der Ausscheidungen, einen Vergleich mit Normalwerten und/oder Vorschläge für den Nutzer, z.B. Ernährungsratschläge oder ähnliches, auszugeben. Zudem kann das Ergebnis vom Auswertprozessor gespeichert und für die Bestimmung von zukünftigen Ergebnissen berücksichtigt werden, und/oder weitere Schritte aufgrund des Ergebnisses durchgeführt werden.

Der Auswertprozessor kann dazu konfiguriert sein, aufgrund von zugeführten Daten, insbesondere einen Trainingsdatensatz mit erfassten Werten, die Multispektraldaten umfassen, und optional weiteren Informationen, zum Beispiel medizinische Informationen über die Personen, z.B. Alter, Größe, Gewicht, Vorerkrankungen o.ä., deren erfasste Werte zugeführt werden, mittels eines oder mehrerer Algorithmen maschinelles Lernen durchzuführen. Somit können Algorithmen entwickelt werden, die eine oder mehrere Parameter zum Gesundheitszustand des Nutzers jeweils nach aktuellen Wissensstand berechnen. Solche Algorithmen können insbesondere die Quantenphysik berücksichtigen.

Der Auswertprozessor kann dafür konfiguriert sein, weiteres maschinelles Lernen optional nach Initialisierung des Systems aufgrund von zusätzlichen oder neuen Gesundheitsinformationen und/oder mit weiteren Trainingsdatensätzen durchzuführen, sodass das System weiterentwickelt werden kann. Insbesondere können somit neue medizinische Erkenntnisse durch maschinelles Lernen in dem System berücksichtigt werden. Zudem kann der Auswertprozessor konfiguriert sein, zu einem anderen Zeitpunkt (z.B. mit jedem weiteren Bestimmen eines oder mehrerer Parameter zum Gesundheitszustand des Nutzers oder nach einer bestimmten Anzahl von Durchgängen des Bestimmens von einem oder mehreren Parametern zum Gesundheitszustand des Nutzers) weitere Schritte von maschinellen Lernen durchzuführen. Somit kann durch maschinelles Lernen die künstliche Intelligenz des Auswertprozessors weiterentwickelt werden.

Das System kann neben der Klobrille und dem Auswertprozessor auch noch eine oder mehrere weitere Displays umfassen. Beispielsweise kann das System ein Display (z.B. einen Monitor) umfassen, auf dem das Ergebnis angezeigt werden kann, der beispielsweise neben der Toilette angebracht werden kann, oder das Display eines mobilen Endgerätes umfassen, dessen Prozessor der Auswertprozessor des Systems ist. Insbesondere kann der Auswertprozessor ein System von mehreren Prozessoren sein, bei denen zum Beispiel einige Prozessoren in einer Cloud befindlich sein können, die beispielsweise die rechenaufwendigen Schritte durchführen können, wohingegen ein Prozessor in der Klobrille oder in einem mobilen Endgerät oder dem Smartphone des Nutzers die Ausgabe und optional Anzeige des errechneten einen oder mehreren Parametern zum Gesundheitszustand des Nutzers durchführen kann.

Der Auswertprozessor kann dazu konfiguriert sein, die Hautfarbe des Nutzers zu erfassen und basierend darauf das Spektrum und/oder die Intensität der Lichtquelle für eine Multispektralkamera zu bestimmen und die Lichtquelle entsprechend anzusteuern, sodass dann die Erfassung von Werten mit dem entsprechenden Spektrum und/oder der entsprechenden Intensität der Lichtquelle durchgeführt werden kann, beispielsweise durch ein Zuschalten von geeigneten Filtern zur Lichtquelle und/oder Anpassung der Intensität der Lichtquelle. Die Erfassung der Hautfarbe des Nutzers kann beispielsweise mittels Sensors zum Erfassen der Hautfarbe des Nutzers oder mittels einer ersten Messung mit der Multispektralkamera erfolgen. Es kann vorteilhaft sein, die Hautfarbe des Nutzers zu erfassen, bevor die tatsächlichen Messungen an den Körper des Nutzers durchgeführt werden, da je nach Pigmentierung der Haut des Nutzers entsprechend verschiedene Intensitäten von Lichtquellen und/oder verschiedene Spektren benötigt werden können, um Messwerte durch die Haut des Nutzers zu gewinnen, beispielsweise Informationen über das Blut im Körper des Nutzers zu finden. Zudem kann Information über die Hautfarbe des Nutzers ermöglichen, entsprechend die Hautfarbe des Nutzers aus den Spektren, die durch die Haut gewonnen werden, herauszurechnen.

Der Auswertprozessor kann auch dazu konfiguriert sein, Störsignale, wie ein oder mehrere Spektren, z.B. das Spektrum von Wasser, das Spektrum von einem oder mehreren Fenstern, hinter denen die Lichtquelle und/oder Multispektralkamera angeordnet sind und/oder das Spektrum des Hintergrunds, z.B. Reflexionen der Toilettenschüssel, aus den Multispektraldaten herauszurechnen (und somit den einen oder die mehreren Parameter zum Gesundheitszustand des Nutzers ohne diese Störsignale zu bestimmen).

Der Auswertprozessor kann dafür konfiguriert sein, basierend auf dem Ergebnis einen Behandlungsschritt durchzuführen.

Die Erfindung umfasst des Weiteren ein Verfahren zum Erfassen von Werten eines Nutzers mittels einer Klobrille mit einer Vorrichtung zum Erfassen von Werten wie zuvor beschrieben und/oder einem System zum Erfassen von Werten eines Nutzers wie zuvor beschrieben. Bei einem solchen Verfahren werden unter Verwendung von Multispektraldaten ein oder mehrere Parameter zum Gesundheitszustand eines Nutzers bestimmt und ein Ergebnis ausgegeben. Alle Schritte, die zuvor beschrieben wurden und im System bzw. mit der Klobrille durchgeführt werden können, insbesondere vom Auswertprozessor, können entsprechend Schritte des Verfahrens sein.

Weitere Aspekte der Erfindung werden anhand der Figuren erläutert. Hierbei zeigt
**Figur 1a** und **1b** eine Klobrille in Ansicht von schräg oben;
**Figur 2a** und **2b** einen Querschnitt durch einen Teil einer Klobrille;
**Figur 3a** und **3b** einen Querschnitt durch eine Toilettenschüssel mit Klobrille;
**Figur 4** ein System, das eine Klobrille umfasst.

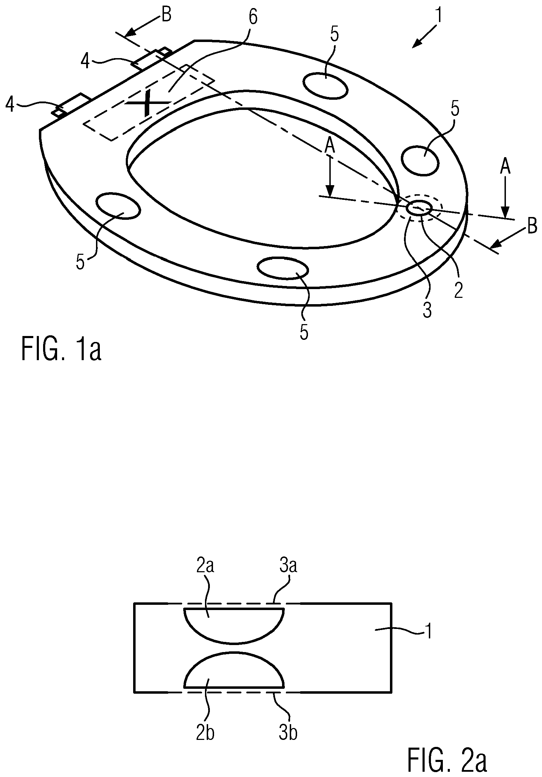

**Figur 1a** zeigt eine Klobrille 1 von schräg oben. Die Klobrille 1 umfasst insbesondere eine Multispektralkamera 2 zum Erfassen von Multispektraldaten (in einer Vorrichtung zum Erfassen von Werten). In dem gezeigten Beispiel ist diese Multispektralkamera 2 hinter einem Fenster 3 in der Klobrille angeordnet und so ausgerichtet, dass sie zum Erfassen eines Spektrums des Körpers des Nutzers ausgerichtet ist. Insbesondere ist die Multispektralkamera 2 so in der Klobrille 1 angeordnet, dass sie typischerweise unter dem Bein eines Nutzers liegt, wenn dieser in normaler Haltung auf der Klobrille 1 sitzt.

Die Klobrille kann einen Anschluss 4 zum Anschließen der Klobrille an eine Toilettenschüssel umfassen, wobei es sich um einen Standardanschluss handeln kann, wie er typischerweise bei normalen Toiletten Einsatz findet, sodass die Klobrille an jede handelsübliche Toilette angeschlossen werden kann. Alternativ können die Anschlüsse passend zu einer bestimmten Toilette ausgebildet sein, die beispielsweise mit der Klobrille vertrieben werden kann, sodass beispielsweise auch eine Stromversorgung und/oder Datenübertragung über die Anschlüsse 4 möglich sein kann (hier nicht gezeigt). Die Klobrille kann weitere Sensoren 5 umfassen, beispielsweise einen oder mehrere der folgenden Sensoren: einen Gassensor, einen Sensor zum Erfassen der Hautfarbe, einen Hautoberflächendetektor, einen Gewichtssensor, einen Temperatursensor, EKG, einen Geigerzähler, einen Biofeedbacksystem. Die Klobrille 1 kann zusätzlich oder alternativ optional ein Laserspektrometer umfassen (nicht gezeigt).

Wie gezeigt, kann die Klobrille 1 optional ein Display 6 umfassen. In der Figur ist das Display 6 beispielhaft am hinteren Ende der Klobrille 1 angeordnet, sodass der Nutzer dies beim Aufstehen und Spülen der Toilette sehen kann. Ebenfalls beispielhaft ist auf dem Display 6 ein binäres Ergebnis in Form eines X angezeigt, das auf eine Abweichung des einen oder mehrerer Parameter zum Gesundheitszustand des Nutzers von den empfohlenen Parametern hinweisen kann. Natürlich können auch andere Ergebnisse, beispielsweise eine andere Form der Warnung, ein binäres o. k. in Form eines Hakens oder eine sonstige andere Form, oder ein detaillierteres Ergebnis, beispielsweise in Form von einem oder mehreren Parametern, Messwerten oder Informationen auf dem Display angezeigt werden.

Von der Klobrille kann optional zusätzlich oder alternativ zur gezeigten Multispektralkamera eine Multispektralkamera umfasst sein, die auf die Ausscheidungen gerichtet sein kann (in Figur 1a nicht sichtbar). In anderen Ausführungsformen kann von der Klobrille eine Multispektralkamera umfasst sein, die auf den Körper des Nutzers und die Ausscheidungen gerichtet werden kann, und beweglich und ansteuerbar, z.B. an der Innenseite der Klobrille 1, angeordnet ist (in Figur 1a nicht gezeigt).

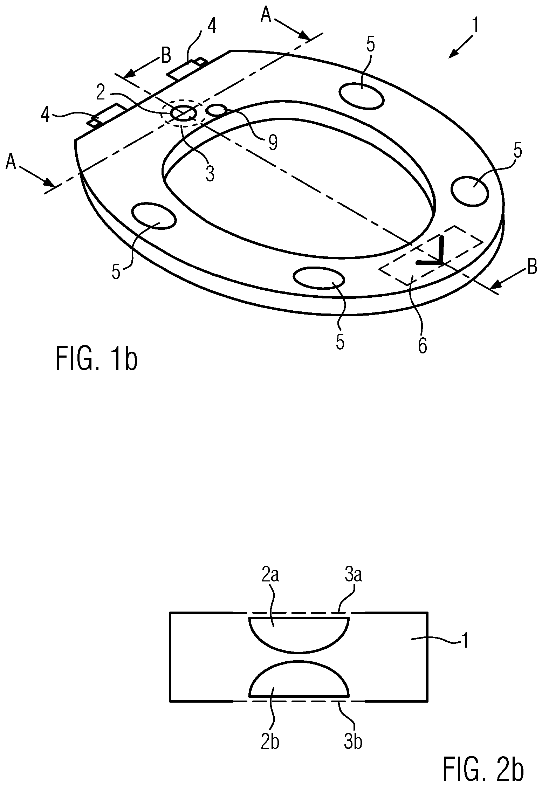

**Figur 1b** zeigt eine alternative Ausführungsform einer Klobrille von schräg oben. In dieser Ausführungsform ist eine Multispektralkamera 2 an der Innenseite des hinteren Bereichs der Klobrille 1 bezüglich der Achse B mittig angeordnet, und ein Display 6 im vorderen Teil der Klobrille 1 bezüglich der Achse B mittig angeordnet. Achse B beschreibt hier die Symmetrieachse der Form der Klobrille (wobei die Vorrichtung zum Erfassen von Werten, insbesondere Multispektralkameras, Sensoren und auch das Display etc. hinsichtlich der Symmetrieachse der Form der Klobrille nicht berücksichtigt werden, also nicht symmetrisch hinsichtlich der Symmetrieachse angeordnet sein müssen). In der gezeigten Ausführungsform ist die Multispektralkamera 2 so angeordnet, dass der Nutzer beim Benutzen der Klobrille normalerweise nicht auf der Multispektralkamera 2 zu sitzen kommt.

In anderen Ausführungsformen kann die Multispektralkamera 2 und/oder das Display 6 auch nicht mittig bezüglich Achse B, sondern seitlich versetzt zu Achse B angeordnet sein (nicht gezeigt).

Wie gezeigt kann die Klobrille 1 auch einen Anschluss 4 zum Anschließen der Klobrille an eine Toilettenschüssel umfassen, der insbesondere wie in Figur 1a beschrieben ausgebildet sein kann. Die Klobrille 1 kann des Weiteren einen oder mehrere weitere Sensoren 5 umfassen, beispielsweise solche Sensoren, wie sie für Fig. 1a beschrieben wurden. Die Klobrille 1 kann optional ein Laserspektrometer umfassen. Optional kann die Klobrille 1 einen Gassensor 9 umfassen, der z.B. in der hinteren Hälfte, z.B. im hinteren Drittel, der Klobrille 1 angeordnet sein kann. Der Gassensor 9 kann beispielhaft an der Innenseite der Klobrille 1 angeordnet sein oder näher an der Innenseite der Klobrille 1 als an der Außenseite der Klobrille.

Auf dem Display 6 ist beispielhaft ein binäres Ergebnis in Form eines Hakens angezeigt, das anzeigen kann, dass der eine oder die mehreren Parameter zum Gesundheitszustand des Nutzers innerhalb der vorgegebenen (normalen) Parameter liegen. Andere Ergebnisse, beispielsweise wie für Figur 1a beschrieben, können natürlich ebenso auf Display 6 angezeigt werden. Wie für Figur 1a beschrieben, kann alternativ oder zusätzlich eine Multispektralkamera umfasst sein, z.B. eine Multispektralkamera, die auf die Ausscheidungen gerichtet ist (in Fig. 1b nicht gezeigt). In anderen Ausführungsformen kann von der Klobrille eine Multispektralkamera umfasst sein, die auf den Körper des Nutzers und die Ausscheidungen gerichtet werden kann, und beweglich und ansteuerbar, z.B. an der Innenseite der Klobrille 1, angeordnet ist (in Figur 1b nicht gezeigt).

**Figur 2a** zeigt einen Querschnitt durch einen Teil einer Klobrille 1, wie er zum Beispiel bei der Klobrille von Fig. 1a durch Linie A aussehen könnte. Dort gezeigt ist eine erste Spektralkamera 2a hinter einem Fenster 3a sowie eine zweite Multispektralkamera 2b hinter einem Fenster 3b.

Multispektralkamera 2a ist so angeordnet, dass sie typischerweise bei Benutzung der Klobrille auf den Körper des Nutzers (hier typischerweise die Unterseite eines Oberschenkels des Nutzers) gerichtet ist. In der gezeigten Multispektralkamera 2a kann eine Lichtquelle integriert sein. Das Spektrum der Lichtquelle kann Spektren umfassen, die zur Untersuchung eines Körpers des Nutzers geeignet sind, insbesondere beispielsweise Infrarotstrahlung, UV-Strahlung oder andere Spektralbereiche, die einen Rückschluss über Körperbestandteile, z.B. Blutbestandteile oder Sauerstoffsättigung des Blutes, erlauben.

Die zweite Multispektralkamera 2b ist beispielhaft hinter Fenster 3b und nach unten ausgerichtet, so dass sie bei Benutzung der Klobrille in Richtung der Toilettenschüssel ausgerichtet ist (und somit Bilder der Ausscheidungen in der Toilettenschüssel aufnehmen kann). Diese Multispektralkamera kann ebenfalls eine integrierte Lichtquelle umfassen, oder eine getrennte Lichtquelle nutzen. Alternativ oder zusätzlich kann sie zur Benutzung mit ohnehin vorhandenem Licht ausgebildet sein, z.B. Umgebungslicht oder Licht einer beleuchteten Toilette.

Fenster 3b kann - wie in Figur 2 gezeigt - im Querschnitt radial durch die Klobrille und die Multispektralkamera 2b näher an der Innenseite der Klobrille als an der Außenseite angeordnet sein, so dass bei Benutzung der Klobrille die Multispektralkamera 2b nicht durch den Rand der Toilettenschüssel verdeckt wird. Fenster 3a ist in Figur 2 im Querschnitt radial durch die Klobrille und die Multispektralkamera 2b ebenfalls zur Innenseite der Klobrille hin versetzt angeordnet. Diese Anordnung kann aber in anderen Ausführungsformen anders sein, beispielsweise kann die Multispektralkamera 2a im Querschnitt durch die Klobrille und die Multispektralkamera 2a mittig zwischen Innen- und Außenseite der Klobrille 1 oder näher an der Außenseite der Klobrille 1 angeordnet sein.

**Figur 2b** zeigt einen Querschnitt durch einen Teil einer Klobrille 1, wie er zum Beispiel bei der Klobrille von Fig. 1b durch Linie A aussehen könnte. Dort gezeigt ist eine erste Spektralkamera 2a hinter einem optionalen Fenster 3a sowie eine zweite Multispektralkamera 2b hinter einem optionalen Fenster 3b.

Die Multispektralkamera 2a und/oder 2b können jeweils so ausgebildet sein, wie für Multispektralkameras 2a und/oder 2b in Figur 2a beschrieben. Alternativ oder zusätzlich (in Figur 2a nicht beschrieben) kann Multispektralkamera 2a zur Benutzung mit ohnehin vorhandenem Licht ausgebildet sein, z.B. mit Umgebungslicht.

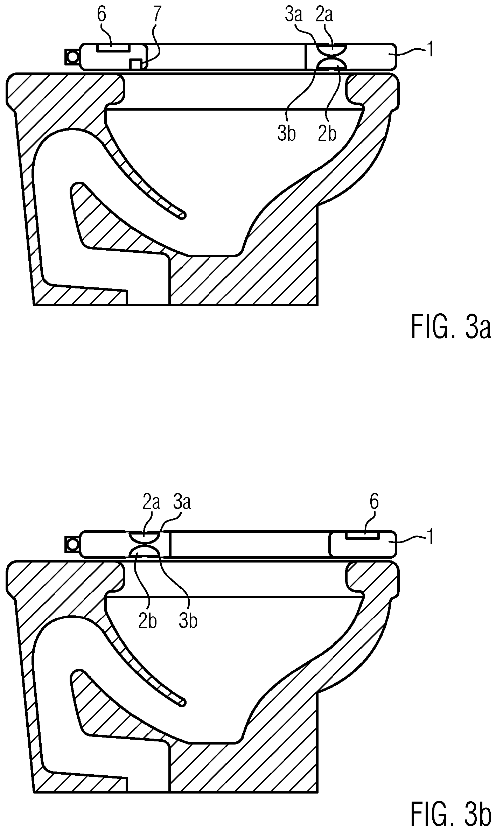

**Figur 3a** zeigt einen Querschnitt durch Multispektralkameras 2 und Klobrille 1, während diese auf einer Toilettenschüssel angeordnet ist. Ein solcher Querschnitt kann beispielsweise ein Querschnitt entlang Linie B in Figur 1a sein. Eingezeichnet sind hierbei beispielhaft zwei Multispektralkameras 2a und 2b, wobei die eine Multispektralkamera 2a, optional hinter Fenster 3a, auf den Körper des Nutzers ausgerichtet ist und die zweite Multispektralkamera 2b, optional hinter Fenster 3b, auf die Ausscheidungen ausgerichtet ist. In dem in Figur 3a gezeigten Beispiel ist optional eine Lichtquelle 7 an einem hinteren Teil der Klobrille 1 vorgesehen, um Licht für die Multispektralkamera 3b bereit zu stellen. Die Lichtquelle 7 kann, z.B. durch mechanische oder elektronische Filter, anpassbare Spektren aufweisen und/oder eine spektrale Verteilung des Lichts aufweisen, die zur Untersuchung von Ausscheidungen abgestimmt ist. Lichtquelle 7 kann eine punktförmige Lichtquelle sein oder ausgedehnt sein, z.B. als Lichtband ausgebildet sein. Eine lichtbandförmige Lichtquelle 7 kann beispielsweise parallel zur Innenseite der Klobrille verlaufen und entlang von mehr als 1 cm, z.B. mindestens 10 cm verlaufen. Eingezeichnet in Figur 3a ist ebenfalls ein optional vorhandenes Display 6 von Klobrille 1, das z.B. wie das Display in Fig. 1a ausgebildet sein kann.

**Figur 3b** zeigt einen Querschnitt durch Multispektralkameras 2a und 2b und Klobrille 1, während diese auf einer Toilettenschüssel angeordnet ist. Ein solcher Querschnitt kann beispielsweise ein Querschnitt entlang Linie B (Achse B) in Figur 1b sein. Die Multispektralkamera 2a kann optional hinter Fenster 3a angeordnet sein und so ausgebildet sein, wie für Multispektralkamera 2a in Figur 3a beschrieben. Multispektralkamera 2b kann optional hinter Fenster 3b angeordnet sein und so ausgebildet sein, wie für Multispektralkamera 3b in Figur 3a beschrieben. Optional kann eine Lichtquelle 7 von der Klobrille 1 umfasst sein, die beispielsweise so ausgebildet sein kann wie für Lichtquelle 7 in Figur 3a beschrieben (nicht gezeigt).

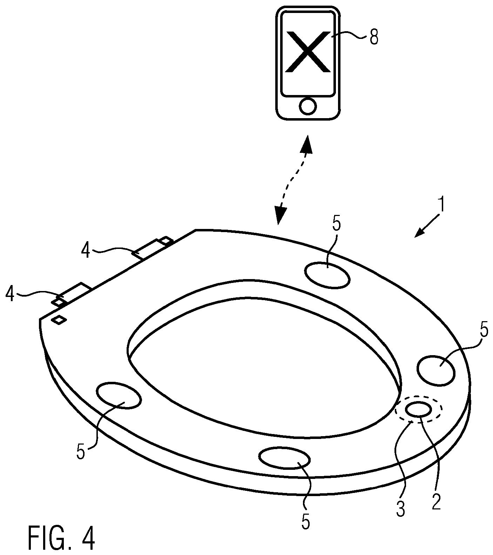

**Figur 4** zeigt ein System, das eine Klobrille 1 umfasst. In dem in Fig. 4 gezeigten System ist eine Klobrille 1 umfasst, bei der die Vorrichtung zum Erfassen von Werten eine Multispektralkamera 2 hinter optionalem Fenster 3 und optional einen oder mehrere Sensoren 5 und/oder ein Laserspektrometer umfassen kann. Zudem ist in dem System auch ein externes Display 8 umfasst, der hier in Form eines mobilen Endgerätes, z.B. Smartphones, gezeigt ist und auf dem ein Ergebnis vom Auswertprozessor ausgegeben werden kann. In anderen Ausführungsformen kann das System ein anderes Display, z.B. einen anderen Monitor, umfassen (in Figur 4 nicht gezeigt). In anderen Ausführungsformen kann im System in der Klobrille 1 die Multispektralkamera 2 (oder mehrere Multispektralkameras, z.B. Multispektralkameras 2a, 2b) an anderen Stellen angeordnet sein, z.B. so angeordnet sein wie in Figur 1b oder Figur 2b gezeigt. Alternativ oder zusätzlich kann von dem System ein Display in der Klobrille umfasst sein (nicht gezeigt).

Der Auswertprozessor in einem solchen System kann beispielsweise den Prozessor des mobilen Endgerätes, z.B. Smartphones, umfassen, der durch eine App dazu konfiguriert ist, ein Ergebnis auszugeben.

In einigen Ausführungsformen kann der Auswertprozessor optional des Weiteren einen oder mehrere weitere/en, z.B. in der Cloud befindliche, Prozessoren umfassen, die dazu konfiguriert sind, weitere Schritte durchzuführen z.B. unter Verwendung von Multispektraldaten ein oder mehrere Parameter zum Gesundheitszustand des Nutzers zu bestimmen. In anderen Ausführungsformen können diese weiteren Schritte auch nur durch den Prozessor des mobilen Endgeräts, z.B. des Smartphones, durchgeführt werden. Es kann nämlich aus Datenschutzgründen vorteilhaft sein, wenn die erfassten Werte, insbesondere Multispektraldaten und optionale weitere von einem oder mehreren Sensoren erfassten Daten, direkt in einem (vom System umfassten) mobilen Endgerät verarbeitet werden können, auf das z.B. nur der Nutzer oder ein kleiner Personenkreis physisch Zugriff hat, und dort das Ergebnis ausgegeben werden kann.

Zusätzlich kann optional in der Klobrille 1 ein Prozessor umfasst sein, der dazu konfiguriert ist, mit der Vorrichtung zum Erfassen von Werten Multispektraldaten und optional weitere Werte, z.B. mit Sensoren 5, zu erfassen. Somit kann in einigen Ausführungsformen der Auswertprozessor nur im mobilen Endgerät und optional der Klobrille angeordnet sein, während er in anderen Ausführungsformen auch in der Cloud oder in einem externen Rechenzentrum befindliche Prozessoren umfassen kann.

## Patentansprüche

1. Klobrille (1) umfassend eine Vorrichtung zum Erfassen von Werten, insbesondere gesundheitsbezogenen Werten eines Nutzers, wobei die Vorrichtung zum Erfassen von Werten eine Multispektralkamera (2, 2a, 2b) zum Erfassen von Multispektraldaten umfasst und die Multispektralkamera in der Klobrille hinter einem Fenster (3, 3a, 3b) angeordnet ist, wobei das Fenster mindestens 80% des Lichts in den Spektralbereichen, in denen nachzuweisende Moleküle typische Spektren aufweisen, passieren lässt, wobei eine Lichtquelle hinter einem anderen Fenster angeordnet ist, und die Multispektralkamera (2, 2b) zum Erfassen von Multispektraldaten der Ausscheidungen und zum Erfassen von Multispektraldaten des Körpers des Nutzers ausgerichtet ist oder ausgerichtet werden kann, und die Vorrichtung zum Erfassen von Werten einen Sensor zum Erfassen der Hautfarbe eines Nutzers und/oder einen Hautoberflächendetektor und/oder ein Laserspektrometer umfasst.

2. Klobrille (1) nach Anspruch 1, wobei die Vorrichtung zum Erfassen von Werten eine zweite Multispektralkamera (2, 2a, 2b) zum Erfassen von Multispektraldaten umfasst.

3. Klobrille (1) nach einem der Ansprüche 1 bis 2, wobei die Vorrichtung zum Erfassen von Werten einen Gassensor umfasst.

4. Klobrille (1) nach einem der Ansprüche 1 bis 3, wobei die Klobrille ein Display (6) umfasst.

5. System zum Erfassen von Werten, insbesondere gesundheitsbezogenen Werten eines Nutzers, umfassend eine Klobrille (1) mit einer Vorrichtung zum Erfassen von Werten nach einem der vorhergehenden Ansprüche und einen Prozessor oder Prozessorsystem, wobei der Prozessor oder das Prozessorsystem dazu konfiguriert ist, unter Verwendung von Multispektraldaten ein oder mehrere Parameter zum Gesundheitszustand des Nutzers zu bestimmen und ein Ergebnis auszugeben.

6. System nach Anspruch 5, wobei der Prozessor oder das Prozessorsystem dazu konfiguriert ist, die Hautfarbe des Nutzers zu erfassen und basierend darauf das Spektrum und/oder die Intensität der Lichtquelle für eine Multispektralkamera zu bestimmen und die Lichtquelle entsprechend anzusteuern.

7. Verfahren zum Erfassen von Werten eines Nutzers mittels einer Klobrille mit einer Vorrichtung zum Erfassen von Werten nach einem der Ansprüche 1 bis 4 und/oder einem System zum Erfassen von Werten nach einem der Ansprüche 5 bis 6, wobei unter Verwendung von Multispektraldaten ein oder mehrere Parameter zum Gesundheitszustand eines Nutzers bestimmt und ein Ergebnis ausgegeben wird.

## Claims

1. Toilet seat (1) comprising a device for detecting values, in particular health-related values of a user, wherein said device for detecting values comprises a multispectral camera (2, 2a, 2b) for collecting multispectral data and the multispectral camera is arranged in the toilet seat behind a window (3, 3a, 3b), wherein the window lets at least 80% of the light pass through in the spectral ranges in which the molecules to be detected have typical spectra, wherein a light source is arranged behind a different window, and said multispectral camera (2, 2b) is oriented or can be oriented to collect multispectral data of the excretions and to collect multispectral data of the body of the user, and said device for detecting values comprises a sensor for detecting the skin color of a user and/or a skin surface detector and/or a laser spectrometer.

2. Toilet seat (1) according to claim 1, wherein said device for detecting values comprises a second multispectral camera (2, 2a, 2b) for collecting multispectral data.

3. Toilet seat (1) according to one of the claims 1 to 2, wherein said device for detecting values comprises a gas sensor.

4. Toilet seat (1) according to one of the claims 1 to 3, wherein said toilet seat comprises a display (6).

5. System for detecting values, in particular health-related values of a user, comprising a toilet seat (1) with a device for detecting values according to one of the preceding claims and a processor or a processor system, wherein said processor or said processor system is configured to determine one or more parameters relating to the state of health of the user using multispectral data and to output a result.

6. System according to claim 5, wherein said processor or said processor system is configured to detect the skin color of the user and based thereupon to determine the spectrum and/or the intensity of the light source for a multispectral camera and to control said light source accordingly.

7. Method for detecting values of a user by way of a toilet seat comprising a device for detecting values according to one of the claims 1 to 4 and/or a system for detecting values according to one of the claims 5 to 6, wherein one or more parameters relating to the state of health of a user is/are determined using multispectral data and a result is output.

## Revendications

1. Siège de toilette (1) comprenant un dispositif de mesure de valeurs, en particulier de valeurs relatives à la santé d'un utilisateur, dans lequel le dispositif de mesure de valeurs comprend une caméra multispectrale (2, 2a, 2b) pour mesurer des données multispectrales et la caméra multispectrale est agencée dans le siège de toilette derrière une fenêtre (3, 3a, 3b), dans lequel la fenêtre laisse passer au moins 80 % de la lumière dans les plages spectrales où les molécules à détecter ont des spectres typiques, dans lequel une source lumineuse est agencée derrière une autre fenêtre, et la caméra multispectrale (2, 2b) servant à mesurer des données multispectrales des excréments et à mesurer des données multispectrales du corps de l'utilisateur est orientée ou orientable, et le dispositif de mesure de valeurs comprend un capteur pour détecter la couleur de peau d'un utilisateur et/ou un détecteur de surface cutanée et/ou un spectromètre laser.

2. Siège de toilette (1) selon la revendication 1, dans lequel le dispositif de mesure de valeurs comprend une deuxième caméra multispectrale (2, 2a, 2b) pour acquérir des données multispectrales.

3. Siège de toilette (1) selon l'une des revendications 1 et 2, dans lequel le dispositif de mesure de valeurs comprend un capteur de gaz.

4. Siège de toilette (1) selon l'une des revendications 1 à 3, dans lequel le siège de toilette comprend un affichage (6) .

5. Système de mesure de valeurs, en particulier de valeurs relatives à la santé d'un utilisateur, comprenant un siège de toilette (1) avec un dispositif de mesure de valeurs selon l'une des revendications précédentes ainsi qu'un processeur ou un système de processeur, dans lequel le processeur ou le système de processeur est configuré pour déterminer un ou plusieurs paramètres de l'état de santé de l'utilisateur en utilisant des données multispectrales et pour générer un résultat.

6. Système selon la revendication 5, dans lequel le processeur ou le système de processeur est configuré pour détecter la couleur de peau de l'utilisateur et, sur la base de celle-ci, pour déterminer le spectre et/ou l'intensité de la source lumineuse pour une caméra multispectrale et commander la source lumineuse en conséquence.

7. Procédé d'enregistrement de valeurs d'un utilisateur à l'aide d'un siège de toilette avec un dispositif de mesure de valeurs selon l'une des revendications 1 à 4 et/ou un système de mesure de valeurs selon l'une des revendications 5 et 6, dans lequel un ou plusieurs paramètres pour déterminer l'état de santé d'un utilisateur sont déterminés en utilisant des données multispectrales et un résultat est généré.
